# EUROPEAN PATENT SPECIFICATION

(11) **EP 1 276 443 B1**
(45) Date of publication and mention of the grant of the patent: **22.03.2006**
(21) Application number: 01920039.3
(22) Date of filing: 04.04.2001
(51) Int. Cl.: A61F 11/08

(54) **EARPLUG**
OHRENSTOEPSEL
BOUCHON D'OREILLE

(30) Priority: 06.04.2000 SE 0001275
(43) Date of publication of application: 22.01.2003
(73) Proprietor: Bacou-Dalloz AB, 260 50 Billesholm (SE)
(72) Inventor: HAEKANSSON, Jörgen, S-282 00 Tyringe (SE); HISELIUS, Per, S-224 68 Lund (SE); PERSSON, Marie, S-255 91 Helsingborg (SE)
(74) Representative: Lind, Urban Arvid Oskar
(86) International application number: PCT/SE2001/000734
(87) International publication number: WO 2001/076520

(56) References cited:
- EP-A1- 0 955 025
- EP-A1- 1 046 382
- GB-A- 1 354 055
- GB-A- 2 075 849
- US-A- 4 540 063
- US-A- 5 113 967
- US-A- 5 488 961
- US-A- 5 881 729

## Description

The present invention relates to the technical field of sound-attenuating earplugs and, in particular, to an earplug of the type which comprises an elongated body of elastic material that is adapted to be inserted into the auditory meatus of an ear. The invention also relates to a method of manufacturing such an earplug. Furthermore, the invention relates to a method of affecting the course of the attenuation curve of such an earplug.

The term "plug" here means a hearing protector which, when being used, is at least partially inserted into the auditory meatus of an ear, unlike ear-muffs which are adapted to be applied on the outside of the ear.

### Background Art

In the technical field of earplugs it is known, in connection with a longitudinal through duct of an earplug, to arrange a membrane in order to reduce the attenuation of the earplug in the range that is the most important one as regards speech perception.

For example, SE 8102931-6 (Racal) discloses that an essentially straight attenuation characteristic up to 2 kHz is aimed at. Said application, which mainly relates to ear-muffs, also shows an earplug having a through duct, in which a membrane is fixed to the duct wall. The membrane functions in the range below its resonance frequency as an attenuation reducing means and, thus, allows more sound to be let through. No detailed discussion of the properties of the membrane is to be found.

Another earplug comprising a membrane is disclosed in US 5881729, which membrane is adapted to allow audible tones to pass therethrough.

A different type of earplug is further disclosed in EP 0955025. This earplug comprises an acoustic filter for attenuation of sound. The acoustic filter consists of a tube containing two rigid disks, each being provided with at least one opening to permit sound waves to travel into and out of the acoustic filter resulting in attenuation of the sound waves.

### Summary of the Invention

The object of the present invention is to provide an improved earplug of the above general type provided with a membrane.

Another object of the invention is to provide an easy method of manufacturing such an earplug.

Yet another object of the invention is to use and affect the sound characteristics of the membrane in a better and more efficient manner.

These objects are achieved by means of an earplug and methods which exhibit the features stated in independent claims 1, 11 and 19.

According to one aspect of the present invention, an earplug is provided, by starting out from a basic plug which has a through duct in the longitudinal direction of the plug. The invention is based on the understanding that the application of the membrane and the possibility of affecting the membrane's own inherent properties and its sound and attenuation affecting properties are considerably facilitated by the membrane being arranged on a stabilising fixing part, a membrane holder, whereby the membrane is applied in the duct. The membrane together with the membrane holder will in the following be named membrane element.

Such a configuration of a membrane element gives great advantages and possibilities regarding a simple, but yet accurate positioning of the membrane in the duct. The membrane holder simply facilitates the handling of the membrane. Since the membrane holder conveniently has a certain degree of stiffness, it may also prestress or tighten the membrane which thus obtains the desired stiffness.

The earplug according to the present invention is preferably a non-disposable plug and is in that case adapted to be used on more than one occasion. Naturally, this makes it necessary for the membrane to be firmly arranged in the duct, in such a manner that there is no internal displacement of or other external action on the membrane in the duct when a user repeatedly removes and inserts the plug. The membrane holder affords this stability and contributes to securing the membrane in place. The membrane holder which preferably has an extended tubular form is with its circumferential surface suitably, in the applied state, in engagement with the wall of the duct.

Advantageously, the membrane element can be arranged in and seal the through duct of the basic plug after the basic plug has been made. An uncomplicated application of the membrane simply means that the membrane element is inserted into the through duct in the longitudinal direction of the plug to a predetermined position, so that the membrane holder engages the duct wall, or means arranged thereon, in order to attach the membrane element. When applying the membrane element in the through duct, the duct is defined or divided, so that one internal and one external duct part are formed.

Due to the design of the membrane element according to the invention there is also a possibility of moulding an earplug round the membrane element by the membrane holder being moulded at or in the duct wall.

According to a preferred embodiment of the invention, the membrane holder has the form of an essentially tubular, preferably circular, cylinder. The membrane is suitably adapted to essentially cover one end of the cylinder.

The membrane and the membrane holder are formed in one piece. Such a membrane element could conveniently, as regards its form, be compared to a mug without an ear or a cartridge case, where the membrane corresponds to the bottom of the mug and the membrane holder corresponds to the cylindrical wall of the mug.

The membrane may be formed as a thin film with a typical thickness of 0.1 mm. The membrane holder may advantageously have a wall thickness of about 0.5 mm.

The membrane element which, after application in the basic plug, in a sealing manner divides the duct into two parts, one internal and one external part, has in a preferred embodiment of the present invention a pure membrane function, i.e. the membrane element is free from further means, such as sound-absorbing means. However, the membrane element may comprise more than one membrane which are held by the membrane holder. According to the invention, the external and internal duct parts of the earplug are preferably completely free from further means, such as further sound-absorbing means. However, it is possible to apply several membrane elements according to the invention in one and the same through duct of the earplug.

A method of efficiently using and affecting the sound affecting characteristics of the membrane and the sound-attenuating characteristics of the earplug is provided by the earplug. No sound is actually "damped" in the earplug by the membrane. Incident sound is simply reflected out again and does not reach the eardrum. The function of the membrane is to "oscillate", which means that some sound goes through, i.e. is not reflected. This function means that the sound attenuation of the ear plug is reduced at a frequency where the membrane oscillates, i.e. at the resonance frequency of the membrane. The effect of the membrane on the attenuation is clearly seen from an attenuation curve of an earplug according to the present invention. The resonance frequency of a membrane is determined, inter alia, by its mass, area, stiffness and prestress.

We have realised that if, for example, it is desirable to provide a membrane with a relatively low resonance frequency, a comparatively thicker membrane may be used. However, the use of a thick membrane has several disadvantages. There is, for example, a risk that the membrane gets too stiff, which then leads to the opposite effect, i.e. higher resonance frequency. However, it is not only the attenuation curve that is directed upwards as regards frequency, but the attenuation also increases at the resonance frequency at issue. If an ideal membrane were made thicker, an increased oscillating mass would be obtained, which would lead to a lower resonance frequency. However, if a *non-ideal* membrane were made thicker, it would give both an increased stiffness and an increased mass, which thus would result in a smaller change of the resonance frequency than for an ideal membrane. An increased stiffness and an increased mass give a decreased sound transmission, i.e. the effect of the resonance is not evident to the same extent.

Furthermore, we have realised that there are great possibilities of affecting the sound characteristics of the membrane by using the air columns which are formed on each side of the membrane when the membrane element has been applied in the through duct and in a sealing manner divides the duct into two parts. Both the air columns can weigh down the membrane and direct its resonance downwards as regards frequency. In other words, a relatively low resonance frequency may be achieved also by means of thin membranes by adapting the length and the area (especially the mouth area) of the duct or the air columns. A long and thin air column is from an acoustic point of view heavier than a short and wide one. For instance, the external air column, i.e. that between the world around and the membrane, may be formed with a narrower inlet hole, which gives a "heavier" column. The internal air column, i.e. that between the eardrum and the membrane, may also be allowed to affect the resonance frequency by different designs of the duct. As regards a duct which is tapering towards the eardrum, the internal air column becomes acoustically heavier than the external air column. The through duct may, of course, be formed in different ways, the acoustic weight being dominated by the narrowest area of the duct and by the length of the duct. Consequently, by choosing the position of the membrane in the through duct and/or the mouth area, e.g. towards the eardrum, it is possible to displace the resonance of the membrane to a suitable frequency. Having a membrane element according to the present invention considerably facilitates the possibility of choosing, as regards the membrane, an accurate position in the duct.

As mentioned above, an earplug according to the present invention is conveniently manufactured by forming a basic plug with a through duct, after which the membrane element is inserted into the duct. This inventive idea gives great freedom of choice and many possibilities of working with different parameters. Since the membrane element is mounted later, it is possible at a late stage of the manufacturing process to determine what properties the earplug should have. It is, for instance, possible to use membranes with various inherent properties independently of the dimensions of the duct. Moreover, it is possible to choose in what direction the membrane element is to be inserted into the duct, i.e. how far into the duct the membrane itself should be placed, etc.

The membrane element according to the present invention has in the preferred cylindrical embodiment suitably a diameter of 2-6 mm, preferably 3-4 mm, for example 3.4 mm. The length of the membrane element is preferably 1-8 mm, for instance 2 mm. The thickness of the membrane itself is preferably 0.005-0.5 mm, such as 0.1 mm, and the wall thickness of the membrane holder itself is preferably 0.3-2 mm, for example 0.6 mm. The membrane element is preferably of a general flexible material which can be adapted to the earplug and the Shore number of the membrane element is preferably 5°-80° A, for instance 60° A.

The membrane element is preferably formed in one piece by silicone injection of LSR (Liquid Silicone Rubber), for example LR 3003 or the like. Silicone injection might be called "reversed" injection moulding. In traditional injection moulding hot thermoplastics are used which are formed, cooled and solidified. However, in silicone injection, one works in a reversed manner by using a cold, liquid silicone fluid which contains a substance that allows the material to be cured when heated. Thus, the liquid, cold silicone fluid is injected into a mould under high pressure, pressed and heated, so that the silicone fluid is cured. By means of this technique, it is possible to mould a membrane which is a thin film of 0.1 mm. The membrane holder is preferably formed to have a wall thickness of about half a millimetre.

The basic plug which is contained in the earplug according to the present invention may, for instance, be manufactured essentially in the same way as the earplug described in EP 0 847 736. The difference is that the duct in the earplug according to the present invention is a through duct and, therefore, the core element round which the plug is moulded is thus made longer so that it extends through the entire cavity in the mould half. The basic plug may either be moulded in one single material or in several materials (e.g. for different plug parts).

In connection with the moulding of the basic plug, the duct wall may be formed so that, when applying the membrane element, it co-operates with the same by the membrane holder engaging the duct wall. The aim of this is to secure the membrane element in place when it has been inserted into the duct. Naturally, such securing can be performed in many ways, for instance by means of a shoulder or by using a mould cavity when forming, which gives an undercut in the duct wall. Thus, a simple snap lock is provided. The membrane element is thus inserted into the duct until it passes the undercut and is locked. There are, of course, also other possible ways of keeping the membrane element in place, such as friction joints, gluing, etc., which all are in the scope of the overall idea of invention. This also includes the possibility of arranging a special retaining means on the duct wall, which is not formed integrally with the wall.

Furthermore, it is possible to make a duct wall with a plurality of such stops, for example shoulders, retaining means etc. in various positions along the duct wall, so that the membrane can be arranged in different positions.

### Brief Description of the Drawings

Fig. 1 is a longitudinal axial section of an earplug according to an embodiment of the present invention.
Fig. 2a shows an enlargement of a portion of the earplug in Fig. 1 with the membrane element.
Figs 2b-2d show examples of a cross-section along the line A-A in Fig. 2a.
Figs 3a-3b schematically show an example of a membrane element for use in an earplug according to the present invention.
Figs 3c-3g illustrate alternative embodiments of the membrane element according to the present invention.
Figs 3h-3i illustrate various cross-sections of the membrane element according to the present invention.
Fig. 4 illustrates how a membrane element is applied in a basic plug according to a preferred embodiment of the invention.
Fig. 5 schematically shows an earplug according to the invention being applied in a user's ear.
Fig. 6 shows, as in Fig. 1, a longitudinal axial section of an earplug according to yet another embodiment of the present invention.
Figs 7a-7c schematically show the principle of a preferred method of manufacturing a membrane element according to the present invention.
Fig. 8 shows an equivalent electric circuit diagram for an earplug according to the present invention.
Figs 9a-9d show diagrams of attenuation curves for earplugs according to the present invention.
Fig. 10 shows a longitudinal axial section of an earplug according to yet another embodiment of the present invention.
Fig. 11 illustrates as Figs 3c-3g an alternative embodiment of the membrane element according to the present invention.

### Detailed Description of Preferred Embodiments

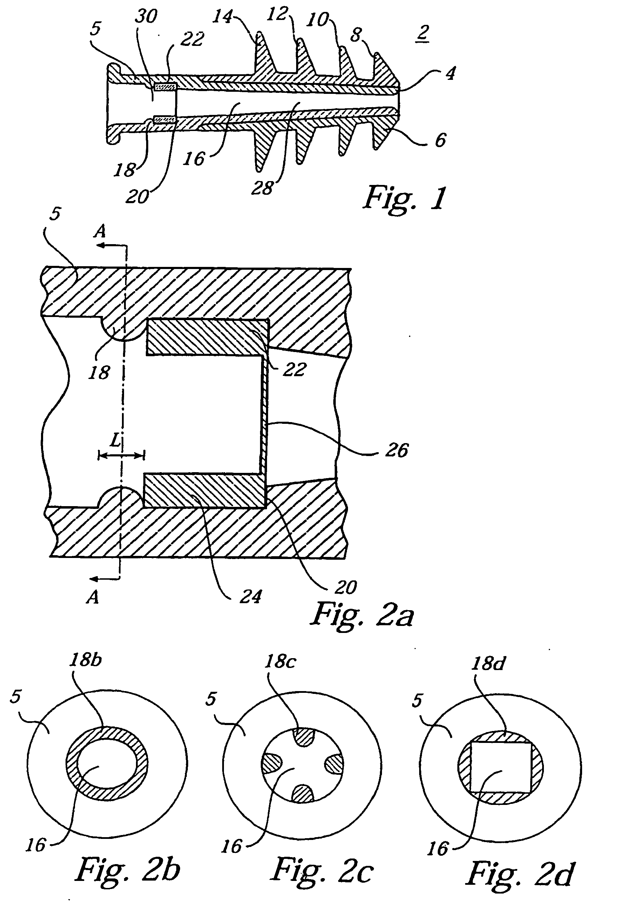

Fig. 1 shows a longitudinal axial section of an earplug 2 according to an embodiment of the present invention. The earplug 2 comprises a core or body part 4 which essentially has the form of a truncated cone. The front part of the core or body part 4 is provided with a surrounding sleeve or sealing part 6. From the circumference surface of the sealing part 6 four integrated annular flanges 8, 10, 12, 14 protrude in the radial direction which is perpendicular to the longitudinal direction of the core or body part 4. A first flange 8 protrudes directly at the front edge of the earplug 2 and has the smallest diameter. The other flanges 10, 12, 14 are evenly distributed over the plug part itself and have diameters that successively increase backwards along the plug 2. The front surface of the flanges 8, 10, 12, 14 is inclined backwards, while the rear surface of the flanges is perpendicular to the longitudinal or axial direction of the plug 2.

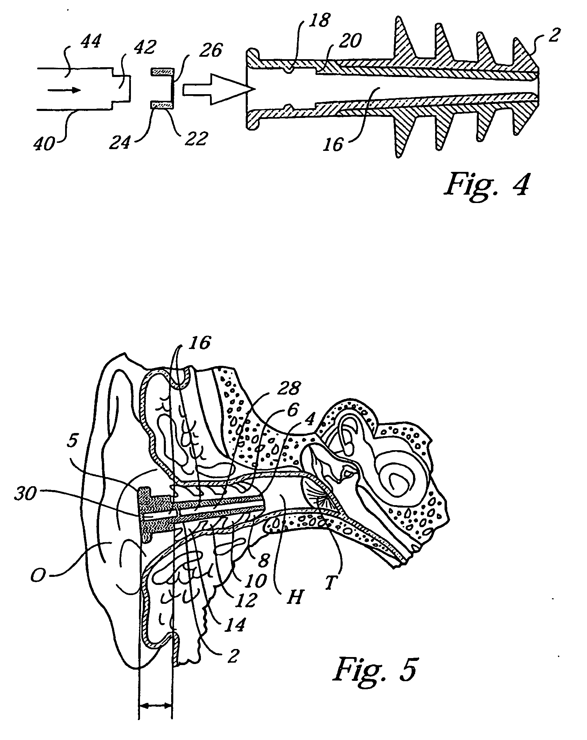

The sleeve-shaped sealing part 6 covers the part of the core or body part 4 which is intended to be inserted into the auditory meatus of the ear, i.e. the entire actual plug. This is illustrated in Fig. 5, where the front part (the sleeve-shaped sealing part 6) of the earplug 2 is inserted into the auditory meatus H of the ear. As seen the four annular flanges 8, 10, 12, 14 abut in sealing condition against the wall of the auditory meatus H. The rear part of the core or body part 4 is adapted to be a handle portion 5. The core or body part 4 has a through axial duct 16 of circular cross-section, the diameter of which decreases approximately from the handle portion 5 to the top. Fig. 1 shows that the duct wall of the core or body part 4 has approximately at the middle of the length of the handle portion 5 an annular bulge 18 and somewhat further forwards an annular protruding shoulder 20 which is formed by decrease of the diameter of the duct. The bulge 18 and the shoulder 20 in the wall are made during the moulding of the core or body part 4 and are formed integrally with the core or body part 4.

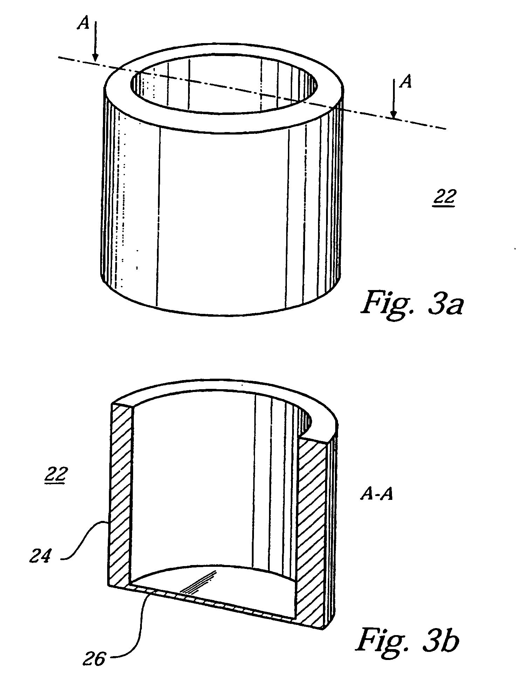

In the through duct 16, a membrane element 22 is applied in a sealing and defining manner between the bulge 18 and the shoulder 20. The portion round the membrane element 22 is shown enlarged in Fig. 2a. The membrane element 22 itself is shown in a perspective view in Fig. 3a, and Fig. 3b is an axial cross-sectional view along the line A-A in Fig. 3a. It is evident from the figures that the membrane element 22 comprises a cylindrical tubular membrane holder 24 with a wall thickness of about 0.5 mm. A circular membrane 26, which is about 0.1 mm thick, is arranged transversely as a lid at the very front of the membrane holder 24. The membrane element 22 is about 2 mm long and has a diameter of about 3.4 mm. The diameter of the membrane is about 2.4 mm. The membrane holder 24 and the membrane 26 are according to this preferred embodiment formed integrally according to a method which will be described below. Figs 1 and 2a show the membrane element 22, as mentioned above, arranged between the bulge 18 and the shoulder 20. The front end of the membrane holder 24 abuts against the annular shoulder 20 protruding from the duct wall, while the rear end of the membrane holder abuts against the bulge 18, and, moreover, the cylindrical surface of the membrane holder engages the duct wall. Thus, the membrane element 22 is fixed. The bulge may have different shapes, for instance annular, or consist of several projections or ribs. This is shown in Figs 2b-2d with examples of a transverse cross-section along the line A-A in Fig. 2a. In Fig. 2b the bulge 18b is annular. In Fig. 2c four projections 18c are shown, but the number of these can, of course, be both greater or smaller, and the shape need not necessarily be rounded. Fig. 2d shows four ribs 18d of which there also may be more or fewer and which may have different shapes. Besides, the length L which is indicated in Fig. 2a may vary for the different types of bulges. The advantage of longer bulges is that the membrane element 22 is very well locked. However, such longer bulges cause greater resistance when inserting the membrane element 22.

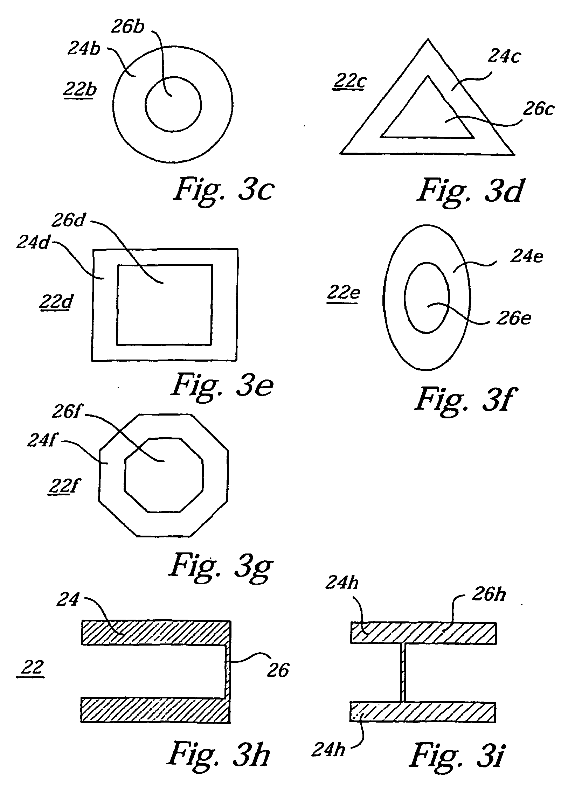

Figs 3c-3g show alternative embodiments of the membrane element 22b-22f for use in an earplug according to the present invention. The membrane elements are seen in the direction of the extension of the duct. Apart from the already shown circular shape, essentially all shapes are possible, both symmetrical and asymmetrical. For example, N-gons may be formed with everything from 3 corners up to an infinite number of corners, i.e. a circular shape. Also various oval forms are possible. In the figures only a few shapes are shown by way of illustration. Fig. 3c shows a circular shape, Fig. 3d shows a triangle, Fig. 3e shows a square shape, Fig. 3f shows an oval shape and Fig. 3g shows an octagon. In all the cases, the membrane 26b-26f constitutes the internal portion and the membrane holder 24b-24f the surrounding external portion. Figs 3h-3i show two possible axially longitudinal sections of the above membrane elements 22b-22f. Naturally, also other cross-sections are possible. All the shown membranes may, for example, have the already shown U-shaped cross-section which is now shown in Fig. 3h, or an H-shaped cross-section as shown in Fig. 3i. In the case of the illustrated H-shaped cross-section, the membrane holder 24h comprises the two parallel legs and the membrane 26h is the transverse leg between these. As shown in Fig. 3i, the membrane 26h is displaced somewhat to the left of the centre of the membrane holder 24h. This H-shaped configuration thus gives the possibility of choosing in an easy way between two different locations of the membrane 26h in the duct and, thus, also air columns which affect the membrane resonance differently. Various types of membrane resonance are obtained simply depending on which end of the membrane element is inserted first into the through duct of the plug. Generally speaking, membrane elements of different shapes can be inserted with either end of the membrane element being directed towards the duct, the final location of the membrane in the through duct determining the appearance of the attenuation curve.

The through duct in the plug conveniently has the same transverse dimension as the membrane element, at least at the portion where the membrane element is placed when using the earplug. For instance, the shoulder against which the membrane element abuts can be formed in accordance with the membrane element. It is essential that a good sealing division of the duct is provided, which results in one internal and one external air column after applying the membrane element, and that the membrane element is firmly fixed.

It is thus evident from the figures that the membrane element 22 divides the through duct 16 into two parts. Between the membrane 26 and the eardrum T (Fig. 5) an internal air column 28 is formed in the duct part in front of the membrane 26 and an air volume in the auditory meatus H from the front end of the earplug 2 to the eardrum T. On the other side of the membrane 26 an external air column 30 is formed in the duct part behind the membrane 26 and the volume of the outside world O, i.e. an infinite volume. The length and the area of the air columns 28, 30 affect the resonance frequency of the membrane 26 as already described.

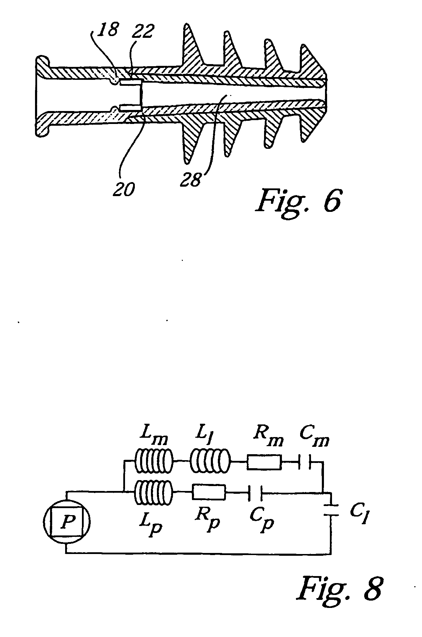

Fig. 6 shows, as Fig. 1, a longitudinal axial section of an earplug according to an embodiment of the present invention. The axial location of the bulge 18 shown in Fig. 6 and the shoulder 20 and, thus, also the membrane element 22 is, however, different from the embodiment according to Fig. 1. The membrane element 22 is now placed further into the duct and, thus, the encased air volume or the air column 28 between the membrane and the eardrum is shorter. The effect of this is that the air column in Fig. 6 does not weigh down the membrane as much as the air column in Fig. 1, whereby the resonance frequency is not displaced to the same extent. It is thus possible, by choosing the location of the membrane in the duct, for example to control the resonance frequency so that, for instance, warning signals at a known frequency is let through more easily or that sound from a machine which is being operated is let through to a greater extent.

The core or the body part and the sealing part may be made of two different materials or in one and the same material, preferably in one piece. As already mentioned, a preferred method of manufacturing these parts is described in EP 0 847 736.

Fig. 4 illustrates the application of a membrane element 22 in the through duct 16 of an earplug 2. According to this preferred method, a membrane element 22 is made separately, as is also the earplug 2 with its core or body part and the sealing part. By means of a piston 40 the cartridge-shaped membrane element 22 is then inserted into the through duct 16 of the earplug 2, as shown by the arrows in the figure, having the membrane 26 at the very front. The earplug 2 is preferably made of a material which is flexible enough to allow the membrane element 22 to be easily inserted. The piston 40 has, as shown in the figure, preferably an outline which supplementary corresponds to the outline of the membrane element 22. A central part 42 which protrudes from the front end of the piston 40 thus fits into the membrane holder 24 and during insertion a circumferential part 44 abuts against the rear edge of the membrane element 22. The membrane element 22 is thus moved forward in the duct 16 and eventually reaches the bulge 18 with its front part (i.e. the membrane 26 and the front part of the membrane holder 24). The membrane element 22 is continually moved forwards with a force enabling its front part to pass the bulge 18. When the front part of the membrane element 22 or membrane holder 24 finally reaches the shoulder 20, the rear part of the membrane holder 24 has passed the bulge 18 and been fixed by snap-in action. In this position the membrane element 22 is thus locked by the membrane holder 24 with its ends abutting against the bulge 18 and the shoulder 20, respectively. As is evident from the figures, the membrane holder 24 is dimensioned so that its transverse dimension essentially corresponds to the dimension of the through duct 16 for retaining of the membrane element 22 when applied in the through duct of the ear plug, while at the same time the duct is sealed.

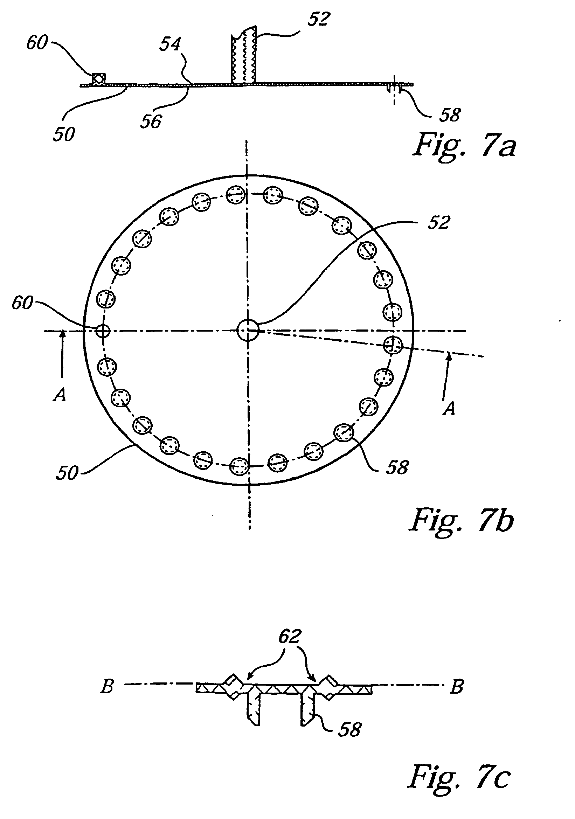

Figs 7a-7c show a preferred method of manufacturing a membrane element 22 according to the present invention. The figures are not to scale, but should only illustrate the manufacturing principle schematically.

Fig. 7a shows a transverse section of a mould 50 and an ingate 52 connected thereto. Fig. 7b illustrates a cross-section along a dividing line of a mould. Fig. 7c shows an enlargement of a portion in Fig. 7a.

As mentioned above, the membrane element 22 is formed preferably by silicone injection of LSR ("Liquid Silicone Rubber"). For example, a silicone rubber from Silopren® LSR series 20xx or the like can be used for the purpose. After the correct composition of the liquid silicone rubber has been obtained, it is transferred from a tube to a screw feeder, alternatively a piston (not shown). By means of the screw feeder the liquid, cold silicone rubber is injected into a mould via an ingate 52 (Fig. 7a). An injection moulding pressure of 50-150 bar is generally enough for LSR. The pressure depends on the cross-section of the feeding duct.

The purpose of the mould 50 is to receive the silicone rubber in its mould cavity, spread, form and cure it, whereby the silicone rubber will be brought to a solid state, after which the ready material may be taken out of the mould 50. Fig. 7a shows the mould 50 in a section along the line A-A in Fig. 7b. The mould 50 according to this preferred embodiment comprises two mould parts: one upper part 54 and one lower part 56, which form a circular mould cavity. Fig. 7b shows a cross-section along the parting line of the mould 50, i.e. the border between the two parts. The ingate 52 is connected to the centre of the circular mould 50 and in the circumference of the mould 50 the mould cavity comprises membrane cavities 58 which together with a guiding pin 60 form a ring. The guiding pin 60 which is also shown in Fig. 7a can be used as an aid for positioning in connection with subsequent handling of the mould product. When the liquid silicone rubber is fed via the ingate 52 to the mould cavity, the silicone rubber will flow out over the entire circular area and also down into the membrane cavities 58. By forming the membrane elements in the periphery of the circular mould cavity, an even distribution of the liquid silicone rubber is obtained. When the moulding process is finished, a disk is thus obtained, which in the periphery exhibits the membrane element. The membrane elements may be pressed out simultaneously, but can also be pressed out one at a time. An earplug with a through duct can advantageously be placed on the top of a membrane element in such a manner that, when the membrane element is pressed out, it is inserted directly into the plug without any intermediate stages.

In order to facilitate the pressing-out of the membrane elements, the mould 50 is formed in such a way that the mould disk is thin round the membrane element. A flash ridge 62 is indicated by the arrows in Fig. 7c which is an enlargement of the portion round the membrane cavity 58 to the right in Fig. 7a. Moreover, the area immediately adjacent to the flash ridge is thicker than the rest of the surrounding area in order to ensure easy pressing-out of the membrane element. Fig. 7c also shows the parting line between the two parts of the mould by means of a dashed line B-B. Thus, it is shown that essentially the entire membrane element is formed in the lower part of the mould.

The mould 50 is usually heated electrically (in general up to 150-230°C depending on the type of LSR) by using, for instance, immersion heaters or filaments. The liquid silicone rubber is injected into the heated mould. The silicone rubber is cured at moulding temperatures of 170-230°C .

When the injected liquid silicone rubber is heated to a high temperature, it tries to swell and return to the injection nozzle. In order to prevent this, the nozzle is kept at a pressure of 5000 kPA (50 bar) until the liquid in the vicinity has started to cure.

The heating and the subsequent volume increase of the silicone rubber in the mould increase the pressure in the moulding cavity, which may attain about 30 000 kPa (300 bar).

Naturally, there are different types of silicone rubber, some of which (e.g. from the series Silopren® LSR 26xx) are more reactive and, thus, are cured faster. Besides, it is possible to start the heating of the silicone rubber in advance, for instance in the screw feeder, in order to speed up the curing process.

As mentioned in the introductory part of the present specification, it is possible by means of this technique to mould a membrane element, in which the membrane itself is a 0.1 mm thin film, and the membrane holder is given a thickness of about 0.5 mm.

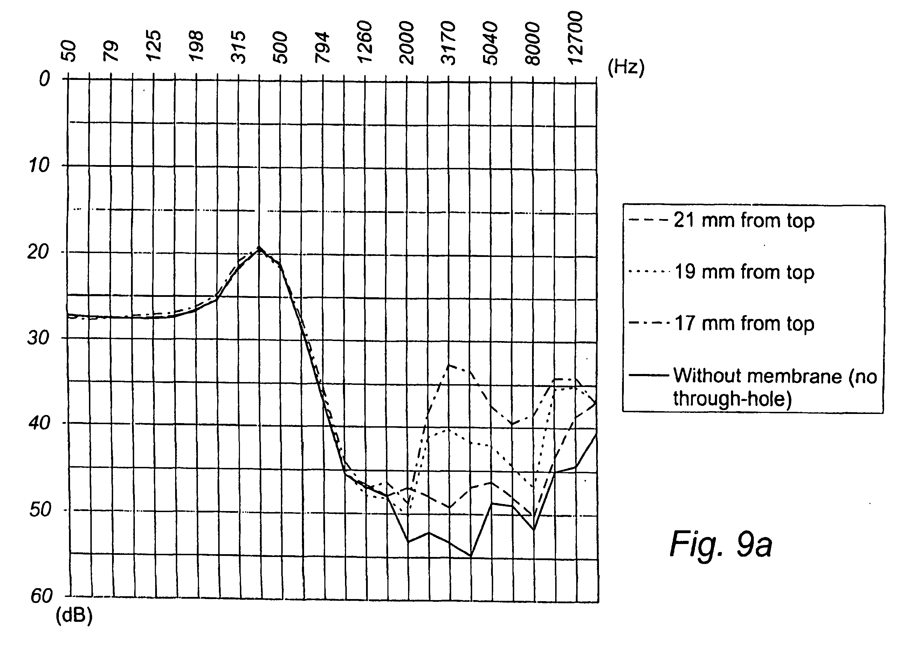

An ordinary open ear, i.e. without a plug inserted, has a natural amplification of sound of about 3 kHz, i.e. the frequency range of human speech. When a plug is inserted, the air volume in the ear is changed, and, therefore, the natural resonance amplification is eliminated or changed, which thus means that the speech perception is impaired. Fig. 8 shows an equivalent electric circuit diagram for an earplug according to the invention, a voltage source P corresponding to the sound pressure that is received, the coil Lₚ corresponding to the acoustic mass of the plug, the capacitor Cₚ corresponding to the acoustic stiffness of the plug, the resistance Rₚ corresponding to the acoustic attenuation of the plug and the capacitor C₁ corresponding to the acoustic stiffness of the included air volume. Furthermore, the coil Lₘ corresponds to the acoustic mass of the membrane, the capacitor Cₘ corresponds to the acoustic stiffness of the membrane and the acoustic attenuation is illustrated by the resistance Rₘ. The acoustic mass of the encased air column corresponds to the coil L₁ which is connected in series with the coil Lₘ. Naturally, also a coil for the external air column may be connected in series with the others, but in this case an equivalent circuit diagram for a plug is shown with a through duct that is tapering inwards (the air columns are of about the same length) and, therefore, the acoustic mass of the thinner air column is predominant.

As known, the impedance of a coil varies with the frequency as jωL and the impedance of a capacitor as 1/jωL. Resistance is independent of the frequency. The acoustic stiffness of the membrane, i.e. the value of the corresponding equivalent capacitor Cₘ, is such that in connection with low frequencies the impedance 1/jωCₘ is greater than the impedance 1/jωCₚ and, thus, the membrane does not at such low frequencies have any considerable effect on the attenuation of the earplug. At high frequencies the impedance jω(Lₘ+L₁) of the coils Lₘ and L₁ (the acoustic mass of the membrane and the encased air column) is predominant, in which case sound at certain frequencies is attenuated to a large extent. Between said low and high frequencies there is a resonance range where the capacitors and the coils co-operate, so that the total impedance gets low and, thus, allows sound to pass. The earplug according to the present invention thus functions as a bandpass filter which lets through sound at frequencies within the predetermined range. It is thus within this range that the resonance is found. By choosing a suitable location of the membrane in the duct, it is possible to obtain a desired air column with a desired acoustic mass, so that the resonance frequency of the membrane is affected. In other words, it is possible to vary the impedance of the coil L₁ and, thus, the attenuation curve of the earplug by choosing the location of membrane. The impedance may also be varied by choosing the cross-sectional area or mouth area of the air column.

Figs 9a-9d show diagrams of attenuation curves for earplugs according to the present invention.

Fig. 9a shows four curves, one of which is for an ordinary earplug without a through duct and a membrane, and the other three are for earplugs according to the invention which have one and the same membrane element (membrane area 3.8 mm² and membrane thickness 1 mm) applied at different distances from the top of the earplug (17, 19 and 21 mm, respectively, from the top). As will be evident from the diagram, the attenuation is high at frequencies above 1000 Hz for an ordinary earplug. By means of an earplug according to the invention which has a membrane element arranged in the through duct of the plug, it is possible to provide a better sound transmission near the frequencies for speech perception. As shown, the attenuation at about 3 kHz is less for the earplugs according to the invention. The curves show that the further away from the top the membrane is placed, the more it is weighed down by a larger air column, which results in the resonance frequency decreasing. The curves further show that the closer to the top the membrane is placed, the better the sound transmission in the frequency range at issue.

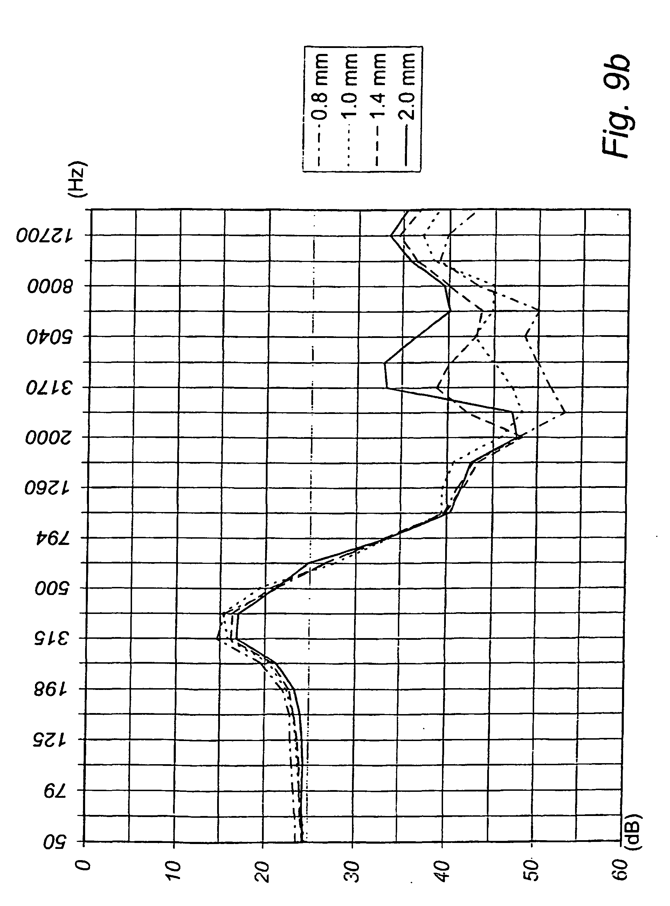

Fig. 9b shows curves for four earplugs with top holes having different areas (diameter = 0.8 mm, 1.0 mm, 1.4 mm and 2.0 mm, respectively). The figure shows that the acoustic mass of the air column increases when the top hole is made smaller, the resonance frequency decreasing and the attenuation increasing. The membrane element is similar to that in Fig. 9a.

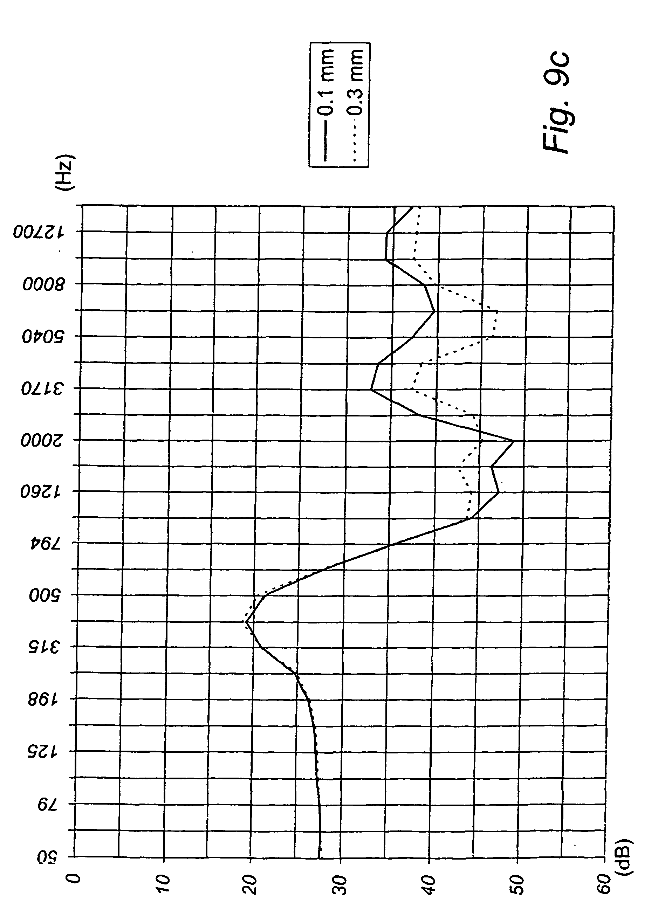

Fig. 9c shows two curves for earplugs, in which membranes of different thickness (0.1 mm and 0.3 mm, respectively) are applied in the same position (17 mm from the top) in the through duct of two similar plugs. By providing a thicker membrane both a greater mass and a greater stiffness of the non-ideal membrane are obtained. The diagram shows that this has no effect as regards frequency but the attenuation is smaller and the sound transmission thus higher for the thinner membrane.

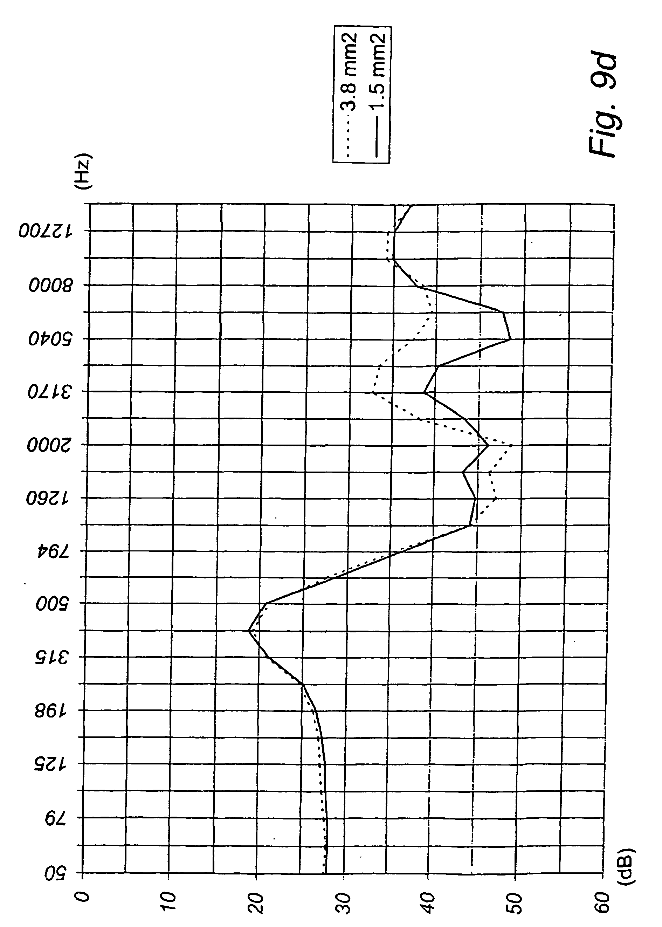

Fig. 9d shows two curves for earplugs, in which the applied membrane has different areas (3.8 mm² and 1.5 mm², respectively). The dimensions of the through duct are the same in both earplugs, and both membranes are arranged 17 mm from the top. As seen, the frequency is not affected to any considerable extent in this case, but the sound transmission is improved by means of the membrane with the greater area.

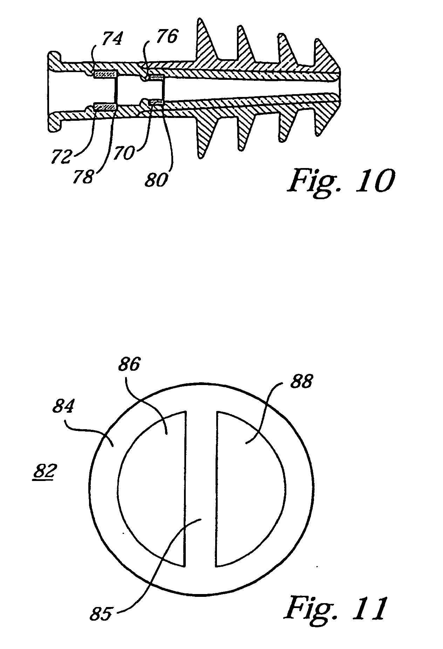

Fig. 10 shows a longitudinal axial section of an earplug according to yet another embodiment of the present invention. This figure illustrates that more than one membrane element can be inserted into the earplug. In this example two membrane elements 70, 72 are inserted, one 70 of which is arranged further into the duct than the other one 72. The membranes are fixed between one bulge 74, 76 and one shoulder 78, 80 each. By means of two membranes which have the same resonance frequency, a resonance is obtained in such a configuration which remains at about the original resonance frequency since both the mass and the stiffness increase. However, the attenuation during the resonance for this double configuration becomes higher by comparison with a plug having one single membrane element.

Naturally, it is also possible, instead of using two membrane elements, to provide a membrane element which comprises a membrane holder, on which two membranes are arranged, one behind the other.

However, Fig. 10 shows the possibility of choosing two different locations of a single membrane element. If one single membrane element is to be used in the earplug in Fig. 10, air columns of different size may be provided (and, thus, various resonance frequencies) depending on between which bulge and shoulder a membrane element is placed.

Fig. 11 illustrates, as Figs 3c-3g, an alternative embodiment of a membrane element according to the present invention. The membrane element 82 is, as in the figures described above, seen in the direction of the extension of the duct. This figure illustrates that the membrane element 82 can comprise several membranes 86, 88 that are arranged next to one another on a membrane holder 84. In this illustrated example, the membrane holder 84 is a circular cylinder (cf. Fig. 3c) which also has a cross-link 85 that extends along the diameter of the cylinder. Consequently, two membranes 86, 88 are provided which are separated by the cross-link 85. If the membranes 86, 88 have different respective resonance frequencies, two resonance peaks are obtained, which makes it possible to decrease the attenuation in a greater frequency range.

The invention is, of course, not limited to the preferred embodiments described above which have been shown by way of example. It should be understood that a plurality of modifications and variations can be provided without abandoning the scope of the present invention which is defined in the appended claims.

## Claims

1. A sound-attenuating earplug (2) which comprises a basic plug with a through duct (16) in the longitudinal direction of the plug, in which duct a membrane (26) extends in a sealing manner in the transverse direction of the duct, thereby forming, when the earplug is applied in an ear, an internal air column on one side of the membrane and/or an external air column on the other side of the membrane, wherein the membrane (26) constitutes a part of a membrane element (22) which further comprises a membrane holder (24) that holds the membrane, the membrane holder being adapted to lock the membrane element in the duct, **characterised in that** the membrane and the membrane holder are formed in one piece, and wherein the duct wall is locally formed to engage the membrane holder (24) in order to lock the membrane element in a predetermined position.

2. An earplug as claimed in claim 1, wherein the membrane and the membrane holder are moulded in one piece, preferably of a flexible material, such as silicone rubber.

3. An earplug as claimed in claim 1, wherein the membrane holder (24) essentially has the form of a tubular cylinder, the circumferential surface of which is engaged with the duct wall, the membrane preferably forming a lid that covers one end of the cylinder.

4. An earplug as claimed in claim 1, wherein the duct wall is locally decreased as regards its diameter in such a manner that a shoulder (20) is formed against which the front end of the membrane holder abuts, and wherein the duct wall preferably also is locally decreased as regards its diameter so that a bulge (18) is formed against which the rear end of the membrane holder abuts.

5. An earplug as claimed in any one of claims 1-3, wherein the duct wall is provided with an attaching means for engaging the membrane holder in order to lock the membrane element.

6. An earplug as claimed in any one of the preceding claims, wherein the membrane element (82) comprises at least one further membrane (88) which is held by the membrane holder.

7. An earplug as claimed in any one of the preceding claims, wherein at least one further membrane element is locked in the duct.

8. An earplug as claimed in any one of the preceding claims, wherein the membrane is adapted to get into resonance in the frequency range for speech perception, preferably in the range of 1 kHz - 4 kHz.

9. An earplug as claimed in any one of the preceding claims, wherein the membrane is adapted to get into resonance in a predetermined frequency range of, for example, warning signals or machine sound.

10. An earplug as claimed in any one of the preceding claims, wherein the membrane element (22) is arranged in the duct (16) at a distance from the ends of the duct, for instance, essentially halfway between the ends of the duct.

11. A method of manufacturing a sound-attenuating earplug (2), comprising the steps of
making a basic earplug having a through duct (16),
making a membrane element (22) which comprises a membrane holder (24) and a membrane (26) that is arranged thereon, and
applying the membrane element in the duct of the basic plug so that the membrane element is held in the duct by the membrane holder, and so that the membrane extends in a sealing manner in the transverse direction of the duct, thereby forming, when the earplug is applied in an ear, an internal air column on one side of the membrane and/or an external air column on the other side of the membrane, **characterised in that** the membrane holder (24) and the membrane (26) are formed in one piece, and wherein the duct wall is locally formed to engage the membrane holder (24) in order to lock the membrane element in a predetermined position.

12. A method as claimed in claim 11, wherein the membrane and the membrane holder are moulded in one piece, preferably of a flexible material, such as silicone rubber.

13. A method as claimed in claim 11, wherein the membrane element (22) is applied in the basic plug by being inserted therein after the basic plug has been provided with its through duct (16).

14. A method as claimed in any one of claims 11-13, wherein the duct (16) is formed in such a manner that a shoulder (20) is provided, which gives a predetermined position for the membrane element to abut against, the membrane element being inserted into the duct towards the shoulder, preferably by means of a piston.

15. A method as claimed in any one of claims 11-14, wherein the duct (16) is formed so that a bulge (18) is provided, which contributes to locking the membrane element when it has been inserted into the duct and passed said bulge, the rear end of the membrane element preferably abutting against the bulge.

16. A method as claimed in any one of claims 11-15, wherein the membrane element (22) is dimensioned so that its transverse dimension corresponds to the transverse dimension of the duct (16) at the duct part where the membrane element is to be applied.

17. A method as claimed in claim 11, wherein the membrane element (22) is applied in the basic plug by the basic plug being mould round the membrane element so that it is kept in position by means of the membrane holder (24).

18. A method as claimed in any one of claims 11-17, wherein the membrane element (22) is arranged in the duct (16) in a position which is separated from the ends of the duct, such as essentially halfway between the ends of the duct.

19. A membrane element (22) adapted to be placed in the through duct (16) of a sound-attenuating earplug (2), the membrane element comprising a membrane (26) and a membrane holder (24) which is adapted to hold the membrane, the membrane holder being dimensioned in such a manner that its transverse dimension essentially corresponds to the transverse dimension of the through duct for retaining of the membrane element in a duct-sealing manner in connection with application in the duct, **characterised in that** the membrane and the membrane holder are formed in one piece, and **in that** the membrane holder is adapted to engage a local formation of the duct wall in order to lock the membrane element in a predetermined position.

20. A membrane element as claimed in claim 19, wherein the membrane and the membrane holder are formed of a flexible material, such as silicone rubber.

21. A membrane element as claimed in claim 19, wherein the shape of the membrane holder essentially corresponds to a hollow cylinder, and the membrane forms a lid which preferably covers one end of the cylinder.

22. A membrane element as claimed in any one of claims 19-21, wherein the membrane element (22) is dimensioned in such a manner that it is afterwards insertable to a predetermined position in the through duct of an existing earplug.

23. A membrane element as claimed in any one of claims 19-22, wherein the membrane holder (84) holds at least one further membrane (88).

## Patentansprüche

1. Schalldämpfender Ohrenstöpsel (2), der einen Basisstöpsel mit einem Durchgangskanal (16) in der Längsrichtung des Stöpsels enthält, wobei sich in dem Kanal eine Membran (26) in einer abdichtenden Weise in der Querrichtung des Kanals erstreckt, wodurch eine innere Luftsäule auf der einen Seite der Membran und/oder eine äußere Luftsäule auf der anderen Seite der Membran ausgebildet wird, wenn der Ohrenstöpsel in ein Ohr eingesetzt wird, wobei die Membran (26) einen Teil eines Membranelements (22) bildet, welches außerdem einen Membranhalter (24) umfasst, der die Membran hält, wobei der Membranhalter eingerichtet ist, das Membranelement in dem Kanal zu befestigen, **dadurch gekennzeichnet, dass** die Membran und der Membranhalter in einem Stück ausgebildet sind und wobei die Kanalwand lokal ausgeformt ist, am Membranhalter (24) anzugreifen, um das Membranelement in einer vorgegebenen Lage zu befestigen.

2. Ohrenstöpsel nach Anspruch 1, wobei die Membran und der Membranhalter in einem Stück, vorzugsweise aus einem flexiblen Material, wie z.B. Silikongummi, ausgeformt sind.

3. Ohrenstöpsel nach Anspruch 1, wobei der Membranhalter (24) im Wesentlichen die Form eines röhrenförmigen Zylinders aufweist, dessen Umfangsfläche an der Kanalwand anliegt, wobei die Membran vorzugsweise eine Kappe ausbildet, die ein Ende des Zylinders abdeckt.

4. Ohrenstöpsel nach Anspruch 1, wobei die Kanalwand hinsichtlich ihres Durchmessers lokal derart verkleinert ist, dass eine Schulter (20) ausgebildet wird, an welche das vordere Ende des Membranhalters anstößt, und wobei die Kanalwand vorzugsweise hinsichtlich ihres Durchmessers auch lokal verkleinert ist, so dass eine Aufwölbung (18) ausgebildet wird, an welcher das hintere Ende des Membranhalters anstößt.

5. Ohrenstöpsel nach einem der Ansprüche 1 - 3, wobei die Kanalwand mit einem Befestigungsmittel zur Aufnahme des Membranhalters versehen ist, um das Membranelement festzuhalten.

6. Ohrenstöpsel nach einem der vorhergehenden Ansprüche, wobei das Membranelement (82) mindestens eine weitere Membran (88) umfasst, die durch den Membranhalter gehalten wird.

7. Ohrenstöpsel nach einem der vorhergehenden Ansprüche, wobei mindestens ein weiteres Membranelement in dem Kanal befestigt ist.

8. Ohrenstöpsel nach einem der vorhergehenden Ansprüche, wobei die Membran eingerichtet ist, im Frequenzbereich fiir Sprachwahrnehmung, vorzugsweise im Bereich von 1 kHz - 4 kHz in Resonanz zu geraten.

9. Ohrenstöpsel nach einem der vorhergehenden Ansprüche, wobei die Membran eingerichtet ist, in einem vorgegebenen Frequenzbereich, zum Beispiel von Warnsignalen oder Maschinenschall, in Resonanz zu geraten.

10. Ohrenstöpsel nach einem der vorhergehenden Ansprüche, wobei das Membranelement (22) in dem Kanal (16) bei einem Abstand von den Enden des Kanals angeordnet ist, zum Beispiel nahezu in der Mitte zwischen den Enden des Kanals.

11. Verfahren zum Herstellen eines schalldämpfenden Ohrenstöpsels (2) mit den Schritten:
Herstellen eines Basisstöpsels, der einen Durchgangskanal (16) aufweist,
Herstellen eines Membranelements (22), das einen Membranhalter (24) und eine Membran (26) umfasst, die darauf angeordnet ist,
Einsetzen des Membranelements in den Kanal des Basisstöpsels, so dass das Membranelement in dem Kanal durch den Membranhalter gehalten wird und so dass sich die Membran in einer abdichtenden Weise in der Querrichtung des Kanals erstreckt, wodurch eine innere Luftsäule auf der einen Seite der Membran und/oder eine äußere Luftsäule auf der anderen Seite der Membran ausgebildet wird, wenn der Ohrenstöpsel in das Ohr eingesetzt wird, **dadurch gekennzeichnet, dass** der Membranhalter (24) und die Membran (26) in einem Stück ausgebildet sind und wobei die Kanalwand lokal ausgeformt ist, am Membranhalter (24) anzugreifen, um das Membranelement in einer vorgegebenen Lage zu befestigen.

12. Verfahren nach Anspruch (11), wobei die Membran und der Membranhalter in einem Stück, vorzugsweise aus einem flexiblen Material, wie z.B. Silikongummi, ausgeformt sind.

13. Verfahren nach Anspruch 11, wobei das Membranelement (22) in den Basisstöpsel eingesetzt ist, indem es dort hinein eingeführt wird, nachdem der Basisstöpsel mit seinem Durchgangskanal (16) versehen wurde.

14. Verfahren nach einem der Ansprüche 11 - 13, wobei der Kanal (16) derart ausgebildet ist, dass eine Schulter (20) bereitgestellt wird , welche für das Membranelement eine vorgegebene Lage ergibt, um daran anzustoßen, wobei das Membranelement in den Kanal vorzugsweise mit Hilfe eines Kolbens zur Schulter hin eingeführt wird.

15. Verfahren nach einem der Ansprüche 11 - 14, wobei der Kanal (16) derart ausgebildet ist, dass eine Aufwölbung (18) bereitgestellt wird, welche zum Befestigen des Membranelements beiträgt, wenn es in den Kanal eingeführt wurde und die Aufwölbung passiert hat, wobei das hintere Ende des Membranelements vorzugsweise an der Aufwölbung anstößt.

16. Verfahren nach einem der Ansprüche 11 - 15, wobei das Membranelement (22) so dimensioniert ist, dass seine Querabmessung mit der Querabmessung des Kanals (16) an dem Kanalteil übereinstimmt, wo das Membranelement einzusetzen ist.

17. Verfahren nach Anspruch 11, wobei das Membranelement (22) in den Basisstöpsel eingesetzt wird, indem der Basisstöpsel um das Membranelement herum so ausgeformt wird, dass es mit Hilfe des Membranhalters (24) ortsfest gehalten wird.

18. Verfahren nach einem der Ansprüche 11 - 17, wobei das Membranelement (22) in dem Kanal (16) an einer Stelle angeordnet ist, die von den Enden des Kanals abgetrennt ist, wie z.B. nahezu in der Mitte zwischen den Enden des Kanals.

19. Membranelement (22), das eingerichtet ist, in dem Durchgangskanal (16) eines schalldämpfenden Ohrenstöpsels (2) angeordnet zu werden, wobei das Membranelement eine Membran (26) und einen Membranhalter (24) umfasst, der eingerichtet ist, die Membran zu halten, wobei der Membranhalter derart dimensioniert ist, dass seine Querabmessung im Wesentlichen mit der Querabmessung des Durchgangskanals übereinstimmt, um das Membranelement in einer Weise festzuhalten, welche bei einem Einsatz im Kanal den Kanal abdichtet, **dadurch gekennzeichnet, dass** die Membran und der Membranhalter in einem Stück ausgebildet sind und dass der Membranhalter eingerichtet ist, an einem lokalen Gebilde der Kanalwand anzugreifen, um das Membranelement in einer vorgegebenen Lage festzuhalten.

20. Membranelement nach Anspruch 19, wobei die Membran und das Membranelement aus einem flexiblen Material, wie z.B. Silikongummi, ausgebildet sind.

21. Membranelement nach Anspruch 19, wobei die Form des Membranelements im Wesentlichen einem Hohlzylinder entspricht und die Membran ein Kappe ausbildet, welche vorzugsweise ein Ende des Zylinders abdeckt.

22. Membranelement nach einem der Ansprüche 19 - 21, wobei das Membranelement (22) derart dimensioniert ist, dass es nachträglich in den Durchgangskanal eines vorhandenen Ohrenstöpsels zu einer vorgegebenen Stelle eingeführt werden kann.

23. Membranelement nach einem der Ansprüche 19 - 22, wobei der Membranhalter (84) mindestens eine weitere Membran (88) hält.

## Revendications

1. Bouchon d'oreille d'atténuation de son (2) qui comprend un bouchon de base avec un conduit traversant (16) dans la direction longitudinale du bouchon, dans lequel conduit une membrane (26) s'étend de manière étanche dans la direction transversale du conduit, formant de ce fait, lorsque le bouchon d'oreille est appliqué à une oreille, une colonne d'air interne sur un côté de la membrane et/ou une colonne d'air externe sur l'autre côté de la membrane, dans lequel la membrane (26) constitue une partie d'un élément de membrane (22) qui comprend, en outre, un porte-membrane (24) qui maintient la membrane, le porte-membrane étant adapté pour verrouiller l'élément de membrane dans le conduit, **caractérisé en ce que** la membrane et le porte-membrane sont formés en une pièce, et dans lequel la paroi du conduit est formée localement pour mettre en prise le porte-membrane (24) afin de verrouiller l'élément de membrane à une position prédéterminée.

2. Bouchon d'oreille selon la revendication 1, dans lequel la membrane et le porte-membrane sont moulés en une pièce, de préférence de matériau flexible, tel que du caoutchouc de silicone.

3. Bouchon d'oreille selon la revendication 1, dans lequel le porte-membrane (24) a, essentiellement la forme d'un cylindre tubulaire, dont la surface circonférentielle est mise en prise avec la paroi du conduit, la membrane formant, de préférence, un couvercle qui couvre une extrémité du cylindre.

4. Bouchon d'oreille selon la revendication 1, dans lequel la paroi du conduit est diminuée localement eu égard à son diamètre d'une manière telle qu'un épaulement (20) est formé contre lequel l'extrémité avant du porte-membrane vient en butée, et
dans lequel la paroi du conduit est également localement diminuée eu égard à son diamètre de sorte qu'une protubérance (18) est formée contre laquelle l'extrémité arrière du porte-membrane vient en butée.

5. Bouchon d'oreille selon l'une quelconque des revendications 1 à 3, dans lequel la paroi du conduit est munie d'un moyen de fixation pour mettre en prise le porte-membrane afin de verrouiller l'élément de membrane.

6. Bouchon d'oreille selon l'une quelconque des revendications précédentes, dans lequel l'élément de membrane (82) comprend au moins une autre membrane (88) qui est maintenue par le porte-membrane.

7. Bouchon d'oreille selon l'une quelconque des revendications précédentes, dans lequel au moins un autre élément de membrane est verrouillé dans le conduit.

8. Bouchon d'oreille selon l'une quelconque des revendications précédentes, dans lequel la membrane est adaptée pour entrer en résonance dans la plage de fréquences pour la perception de la parole, de préférence dans la plage de 1 kHz à 4 kHz.

9. Bouchon d'oreille selon l'une quelconque des revendications précédentes, dans lequel la membrane est adaptée pour entrer en résonance dans une plage de fréquences prédéterminée de, par exemple, des signaux d'avertissement ou un son de machine.

10. Bouchon d'oreille selon l'une quelconque des revendications précédentes, dans lequel l'élément de membrane (22) est disposé dans le conduit (16) à une distance depuis les extrémités du conduit, par exemple, essentiellement à mi-chemin entre les extrémités du conduit.

11. Procédé de fabrication d'un bouchon d'oreille d'atténuation de son (2), comprenant les étapes consistant à :
fabriquer un bouchon d'oreille de base ayant un conduit traversant (16),
fabriquer un élément de membrane (22) qui comprend un porte-membrane (24) et une membrane (26) qui est disposée sur celui-ci, et
appliquer l'élément de membrane dans le conduit du bouchon de base de sorte que l'élément de membrane est maintenu dans le conduit par le porte-membrane, et de sorte que la membrane s'étend de manière étanche dans la direction transversale du conduit, formant de ce fait, lorsque le bouchon d'oreille est appliqué à une oreille, une colonne d'air interne sur un côté de la membrane et/ou une colonne d'air externe sur l'autre côté de la membrane, **caractérisé en ce que** le porte-membrane (24) et la membrane (26) sont formés en une pièce, et dans lequel la paroi du conduit est formée localement pour mettre en prise le porte-membrane (24) afin de verrouiller l'élément de membrane à une position prédéterminée.

12. Procédé selon la revendication 11, dans lequel la membrane et le porte-membrane sont moulés en une pièce, de préférence d'un matériau flexible, tel que du caoutchouc de silicone.

13. Procédé selon la revendication 11, dans lequel l'élément de membrane (22) est appliqué dans le bouchon de base en étant inséré dans celui-ci après que le bouchon de base ait été fourni avec son conduit traversant (16).

14. Procédé selon l'une quelconque des revendications 11 à 13, dans lequel le conduit (16) est formé de manière telle qu'un épaulement (20) est prévu, ce qui procure une position prédéterminée pour l'élément de membrane pour venir en butée contre celui-ci, l'élément de membrane étant inséré dans le conduit vers l'épaulement, de préférence au moyen d'un piston.

15. Procédé selon l'une quelconque des revendications 11 à 14, dans lequel le conduit (16) est formé de sorte qu'une protubérance (18) est prévue, qui contribue à verrouiller l'élément de membrane lorsqu'il est inséré dans le conduit et a passé ladite protubérance, l'extrémité arrière de l'élément de membrane venant, de préférence en butée contre la protubérance.

16. Procédé selon l'une quelconque des revendications 11 à 15, dans lequel l'élément de membrane (22) est dimensionné de sorte que sa dimension transversale correspond à la dimension transversale du conduit (16) au niveau de la partie du conduit où l'élément de membrane doit être appliqué.

17. Procédé selon la revendication 11, dans lequel l'élément de membrane (22) est appliqué dans le bouchon de base par le bouchon de base étant moulé de manière arrondie à l'élément de membrane de sorte qu'il est maintenu en position au moyen du porte-membrane (24).

18. Procédé selon l'une quelconque des revendications 11 à 17, dans lequel l'élément de membrane (22) est disposé dans le conduit (16) à une position qui est séparée des extrémités du conduit, telle qu'essentiellement à mi-chemin entre les extrémités du conduit.

19. Elément de membrane (22) adapté pour être placé dans le conduit traversant (16) d'un bouchon d'oreille d'atténuation de son (2), l'élément de membrane comprenant une membrane (26) et un porte-membrane (24) qui est adapté pour maintenir la membrane, le porte-membrane étant dimensionné de manière telle que sa dimension transversale correspond essentiellement à la dimension transversale du conduit traversant pour retenue de l'élément de membrane de manière étanche au conduit en liaison avec l'application dans le conduit,
**caractérisé en ce que** la membrane et le porte-membrane sont formés en une pièce, et **en ce que** le porte-membrane est conçu pour mettre en prise une formation locale de la paroi du conduit afin de verrouiller l'élément de membrane à une position prédéterminée.

20. Elément de membrane selon la revendication 19, dans lequel la membrane and le porte-membrane sont formés d'un matériau flexible, tel que du caoutchouc de silicone.

21. Elément de membrane selon la revendication 19, dans lequel la forme du porte-membrane correspond essentiellement à un cylindre creux, et la membrane forme un couvercle qui couvre de préférence une extrémité du cylindre.

22. Elément de membrane selon l'une quelconque des revendications 19 à 21, dans lequel l'élément de membrane (22) est dimensionné de manière telle qu'il est insérable par la suite à une position prédéterminée dans le conduit traversant d'un bouchon d'oreille existant.

23. Elément de membrane selon l'une quelconque des revendications 19 à 22, dans lequel le porte-membrane (84) maintient au moins une autre membrane (88).
